# FASCICULE DE BREVET EUROPEEN

(11) **EP 0 251 933 B1**
(45) Date de publication et mention de la délivrance du brevet: **28.04.1993**
(21) Numéro de dépôt: 87401554.8
(22) Date de dépôt: 02.07.1987
(51) Int. Cl.: A61K 39/00

(54) **Peptide présentant des propiétés antigéniques isolé du groupe de protéines 28 kd de s. mansoni et son procédé d'isolement, anticorps monoclonaux reconnaissant au moins un épitope dudit peptide et applications de celui-ci dans induction de la synthèse d'anticorps neutralisants**
Antigenisches Peptid der 28KD Proteingruppe aus S. Mansoni und sein Verfahren zur Isolierung, monoklonale Antikörper, die mindestens ein Epitop dieses Peptids erkennen und Verwendungen für die Induzierung der Synthese von neutralisierenden Antikörpern
Peptide having antigenic properties isolated from the S. Mansoni 28KD proteingroup and its isolation process, monoclonal antibodies recognizing at least one epitope of that peptide, and uses in the induction of neutralizing antibodies synthesis

(30) Priorité: 03.07.1986 FR 8609663
(43) Date de publication de la demande: 07.01.1988
(73) Titulaire: INSTITUT PASTEUR DE LILLE, F-59019 Lille Cédex (FR); INSTITUT PASTEUR, F-75724 Paris Cédex 15 (FR); INSTITUT NATIONAL DE LA SANTE ET DE LA RECHERCHE MEDICALE (INSERM), 75654 Paris Cédex 13 (FR)
(72) Inventeur: Balloul, Jean-Marc, F-59000 Lille (FR); Pierce, Raymond, F-59113 Seclin (FR); Grzych, Jean-Marie, F-59700 Marq en Baroeul (FR); Capron, André, F-59133 Phalempin (FR)
(74) Mandataire: Ores, Irène

(56) Documents cités:
- FR-A- 2 268 532
- FR-A- 2 447 194
- US-A- 4 396 600
- Mol. Biochem. Parasitology, vol. 17, pp. 105-114, 1985

## Description

La présente invention est relative à un peptide isolé du groupe de protéines 28 KD de S. mansoni, qui porte un ou des épitopes et à son procédé d'isolement, ainsi qu'à des anticorps monoclonaux aptes à reconnaître les fractions antigéniques peptidiques ainsi isolées et à des compositions thérapeutiques propres à induire la synthèse d'anticorps neutralisants, lesquelles compositions comprennent ledit peptide seul ou en association avec d'autres substances appropriées.

BALLOUL et Al. [MOLECULAR AND BIOCHEMICAL PARASITOLOGY, 17 (1985) p. 105-114] ont décrit la synthèse in vitro d'un antigène constitué par un polypeptide présentant un poids moléculaire de 28 KD et un point isoélectrique compris entre 6,3 et 6,8, qui constitue un produit de traduction in vitro d'ARN total de Schistosoma mansoni.

Les Auteurs ont poursuivi leurs recherche dans le but d'isoler à partir de ce polypeptides de 28 KD, et d'identifier l'épitope ou les épitopes peptidiques responsables des propriétés antigéniques.

Il est connu d'analyser les peptides qui constituent des protéines isolées de gels de SDS, par digestion partielle desdites protéines par une protéase dans un tampon contenant du SDS; on obtient des produits de digestion partielle stables, qui se composent de nombreux peptides dont les poids moléculaires sont suffisemment élevés pour qu'il soit possible de les séparer en gels de SDS acrylamide à 15% [CLEVELAND et Al., THE JOURNAL OF BIOLOGICAL CHEMISTRY, 252, p. 1102-1106, (1977)].

Les Inventeurs ont cherché à isoler, à partir de groupe de protéines 28 KD de S. mansoni, le ou les peptides portant l'activité antigénique anti-Schistosome que présente la protéine 28 KD, et ont fait application dans ce but des techniques d'isolement de fragments peptidiques par protéolyse décrits dans l'Art antérieur.

La présente invention a pour objet un procédé d'isolement d'un peptide portant au moins un épitope, par protéolyse, caractérisé en ce que l'on soumet les protéines 28 KD de S. mansoni à l'action de la protéase V8 en tampon Tris-HCl pH 6,8 contenant du SDS, en ce que la réaction de protéolyse est arrêtée par addition d'un mélange de 2-mercaptoéthanol et de SDS, puis on porte à ébullition à 100° C pendant un temps bref, pour recueillir un peptide dont on vérifie l'activité antigénique à l'aide des anticorps d'un sérum polyclonal ou d'anticorps monoclonaux.

Selon un mode de mise en oeuvre préféré du procédé conforme à la présente invention, la digestion enzymatique de la protéine 28 KD par la protéase V8 est réalisée pendant 30 minutes à 30°C.

Selon un autre mode de mise en oeuvre préféré du procédé conforme à la présente invention, le mélange utilisé pour arrêter la réaction de protéolyse comprend du 2-mercaptoéthanol à raison de 1 µl pour 3 µl de SDS à 10%.

Selon encore un autre mode de mise en oeuvre préféré du procédé conforme à la présente invention, le tampon Tris-HCl dans lequel est dissoute la protéine 28 KD est un tampon 100 mM à 125 mM et contient de 0,08 à 0,12% de SDS.

Selon un autre mode de mise en oeuvre préféré du procédé conforme à l'invention, la vérification de l'activité antigénique du peptide isolé par protéolyse, qui est réalisée par "Western blotting" à l'aide d'un sérum polyclonal de rat, de lapin ou analogue, anti-28 KD, révèle, à l'aide d'anticorps marqués par la peroxydase, dirigés contre des immunoglobulines de rat, de lapin, de souris ou analogue, un épitope peptidique de 6 KD.

Selon encore un autre mode de mise en oeuvre préféré du procédé conforme à l'invention, la vérification de l'activité antigénique du peptide isolé par protéolyse, qui est réalisée par "Western blotting" à l'aide d'un anticorps monoclonal anti-28 KD révèle un épitope peptidique de 8 KD.

La présente invention a pour objet un fragment peptidique, caractérisé en ce qu'il est isolé du groupe de protéines 28 KD de S. mansoni, par protéolyse ménagée, en ce qu'il présente un poids moléculaire de 6 KD et en ce qu'il est reconnu par du sérum polyclonal de lapin, de rat ou analogue, anti-28 KD.

La présente invention a également pour objet un fragment peptidique, caractérisé en ce qu'il est isolé de la protéine 28 KD de S. mansoni par protéolyse ménagée, en ce qu'il présente un poids moléculaire de 8 KD et en ce qu'il est reconnu par un anticorps monoclonal anti-28 KD, d'isotype IgG2a (W 2 AD 12).

La présente invention a de plus pour objet un hybridome dénommé M 5 BD 9, qui fournit des anticorps monoclonaux d'isotype IgM qui reconnaissent l'antigène de 28 kDa.

La présente invention a également pour objet un hybridome dénommé W 2 AD 12, déposé à la CNCM le 9 Mai 1986 sous le n° I-553, qui fournit des anticorps monoclonaux d'isotype IgG2a qui reconnaissent une bande correspondant à un fragment peptidique de 8 KD, isolé par protéolyse ménagée du polypeptide 28 KD de S. mansoni.

Conformément à la présente invention, les hybridomes M 5 BD 9 et W 2 AD 12 sont obtenus en procédant comme suit :
Les anticorps monoclonaux dirigés contre le groupe de protéines de 28 kDa de Schistosoma mansoni ont été obtenus par hybridation cellulaire homologue rat x rat utilisant la souche myelomateuse de rat LOU IR983F [Basin et coll. 1980 Ann. Immunol. (Institut Pasteur) 131 D : 359], et des cultures spleniques de rat LOU immunisés par 50 µg du groupe de protéines de 28 kDa de S. mansoni, en présence d'adjuvant complet de Freund, en deux injections sous-cutanées de 25 µg, à quinze jours d'intervalle.
Après fusion, les cellules productrices d'anticorps anti-28 kDa ont été sélectionnées par des tests radioimmunologiques sur plaques. Un homogenat de vers adulte est fixé sur des plaques de polyvinyle, puis mis en contact avec les anticorps présents dans les surnageants des cultures d'hybrides. La liaison antigène-anticorps est ensuite révélée par addition d'un second anticorps marqué à l'iode ¹²⁵I, dirigé contre les IgG de rat. Les cellules positives dans ce test ont été clonées par la méthode de la dilution limite.

La présente invention a, en outre, pour objet une composition vaccinante contre S. mansoni qui est caractérisée en ce qu'elle contient au moins un fragment peptidique isolé de 28 KD de S. mansoni par protéolyse ménagée, associé à un véhicule approprié.

Selon un mode de réalisation avantageux de la composition vaccinante conforme à la présente invention, celle-ci contient au moins un fragment peptidique de 6 KD et/ou de 8 KD, associé à un véhicule approprié.

Selon un autre mode de réalisation avantageux de la composition vaccinante conforme à la présente invention, celle-ci contient au moins un fragment peptidique isolé de 28 KD de S. mansoni par protéolyse ménagée, associé à l'épitope de l'antigène 38 KD, ainsi qu'à un véhicule approprié.

L'épitope de l'antigène de PM 38 KD est un oligosaccharide qui fait l'objet de la Demande de Brevet français n^{o} 86 06281 du 30 Avril 1986 au nom des Demanderesses.

Outre les dispositions qui précèdent, l'invention comprend encore d'autres dispositions, qui ressortiront de la description qui va suivre.

L'invention sera mieux comprise à l'aide d'un exemple de mise en oeuvre du procédé conforme à la présente invention et d'exemples de caractérisation de l'activité antigénique du peptide conforme à l'invention.

Il doit être bien entendu, toutefois, que cet exemple de mise en oeuvre, est donné uniquement à titre d'illustration de l'objet de l'invention, dont il ne consitue en aucune manière une limitation.

### EXEMPLE 1 - PREPARATION DU PEPTIDE ISOLE du groupe de protéines 28 KD DE S. MANSONI

### Etape I : Préparation de l'antigène de 28 KD de S. Mansoni

Des vers adultes de S. mansoni (souche portoricaine) collectés par perfusion de la veine porte chez des hamsters dorés, ont été homogénéisés dans du tampon PBS dans un homogénéiseur POTTER-ELVEHSEM et l'homogénéisat à été centrifugé à 5000 tours/minute pendant 20 minutes.
Environ 2 mg d'antigènes ont été fractionnés sur des gels de polyacrylamide à 13% en plaques, en utilisant le système tampon discontinu de Laemmli [décrit par LAEMMLI, NATURE (1970), 227, p. 680-685].
Après électrophorèse, les gels ont été colorés par du bleu brillant de COOMASSIE. Les bandes colorées ont été découpées au scalpel et transférées dans un gel d'élution constitué par des coulées de gel de support.
L'élution a été effectuée à 40-60 V pendant 6 à 12 heures. La vitesse de l'élution est fonction du pourcentage du gel qui se sépare et de la dimension moléculaire des protéines que l'on élue. La migration des échantillons a été stoppée à l'interface des couches de NaCL 2M-glycérol.
Lorsque tous les peptides ont été élués dans la couche de glycérol, l'électrophorèse a été stoppée et les échantillons ont été retirés à l'aide d'une pipette PASTEUR. Les échantillons ont alors été dialysés contre de l'eau pour éliminer le SDS et les sels, avant d'être concentrés par lyophilisation. Les protéines fractionnées ont alors été réanalysées sur du gel de polyacrylamide à 13%, en plaque.
Les protéines de PM 28 KD obtenue par la technique de fractionnement qui vient d'être décrite, et qui sont caractérisées en ce qu'elles induit sent une réponse d'anticorps et en ce que leur antisérum immunoprécipite les antigènes 28 KD correspondants parmi les produits de traduction in vitro aussi bien d'ARN de vers adulte et d'ARN de Schistosomules de S. mansoni, sont retenues pour être traitées conformément à l'Etape II ci-dessous.

### Etape II: Isolement par protéolyse d'un peptide des protéines 28 KD de S. mansoni

10 µg de protéines de 28 KD sont dissous dans 30 µl de tampon 125 mM Tris-HCl (pH 6,8) contenant 0,1% de dodécysulfate de sodium (SDS).
On ajoute 1 µg de protéase V8 et la digestion enzymatique de l'antigène de 28 KD est réalisée à 37°C pendant 30 minutes, au bain-marie.
La réaction est arrêtée par l'addition de 1 µl de 2-mercaptoéthanol et de 3 µl de SDS à 10%. L'ensemble est porté à 100°C au bain-marie durant 6 minutes.
On recueille des échantillons constitués par un peptide dont on détermine les propriétés antigéniques.

### EXEMPLE 2 - ANALYSE DE LA REPONSE ANTIGENIQUE DU PEPTIDE DE L'EXEMPLE 1

10 µg du peptide obtenu à l'Exemple 1 sont déposés sur un gel de polyacrylamide à 20%. Le peptide ainsi fractionné est transféré sur une feuille de nitrocellulose comme décrit par TOWBIN et Al. [1984, J. Immunol. Methods 72, p. 471].
La feuille de nitrocellulose est tout d'abord saturée par une solution de BSA 3% tamponnée par du phosphate mono- et disodique 10 mM pH 7,2 contenant 0,15 M NaCl - 50 µl d'anticorps, lesquels anticorps sont soit des anticorps d'un sérum polyclonal de rat ou de lapin, soit des anticorps d'ascite monoclonale, soit des anticorps obtenus à partir d'un hybridome, spécifiquement d'hybridome W 2 AD 12, M 5 BD 9.
Antigène et anticorps sont laissés en contact durant une nuit à température ambiante, pour obtenir un "Western blot" qui est lavé trois fois trente minutes en tampon phosphate.

Des anticorps marqués par la peroxydase (fournis par PASTEUR PRODUCTION) dirigés soit contre des immunoglobulines de lapin (dans le cas des anticorps polyclonaux), soit contre des immunoglobulines de rat (dans le cas des anticorps monoclonaux), sont ensuite ajoutés et incubés avec le "blot" durant 2 heures. Trois nouveaux lavages sont réalisés et la présence des immuno-complexes est révélée par l'addition d'une solution contenant 20% de méthanol froid, 30 mg de 4-chloro-1-naphtol, 0,1% d'H₂O₂ pour 100 ml de tampon phosphate, 0,15 M NaCl.

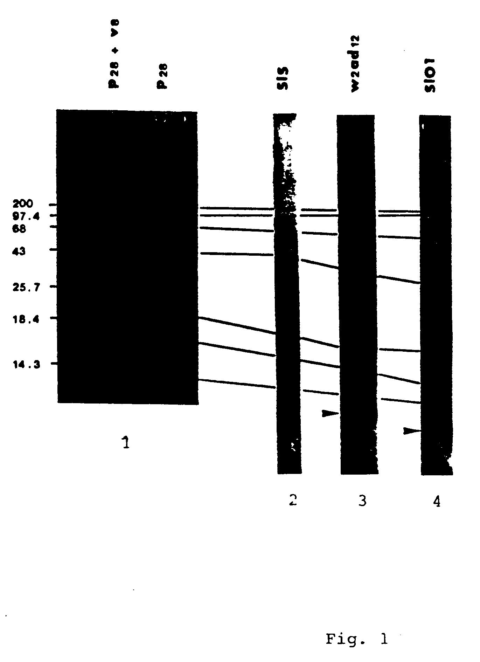

Le "Western blotting" est représenté à la Figure 1 annexée dans laquelle :
- le bloc 1 représente dans sa partie inférieure le peptide isolé par protéolyse, conforme à l'invention et à sa partie supérieure les protéines 28 KD;
- le bloc 2 représente le "blot" obtenu avec un sérum de lapin sain;
- le bloc 3 représente le "blot" obtenu avec des anticorps monoclonaux provenant de l'hybridome W 2 AD 12;
- le bloc 4 représente le "blot" obtenu avec du sérum polyclonal de lapin anti-28 KD.

Ce "Western blotting" montre que l'anticorps monoclonal W 2 AD 12 porteur d'une activité cytotoxique vis-à-vis de la larve post-infestante de Schistosoma mansoni reconnaît un fragment de 8 kDa dérivé des protéines de 28 kDa.

La reconnaissance par un anticorps de classe anaphylactique suggère l'importance de ce peptide dans l'induction d'une réponse protectrice vis-à-vis de S. mansoni.

L'épitope porté par un fragment de 6 kDa, reconnu par le sérum de lapin n'est pas reconnu par l'anticorps monoclonal de rat suggérant que la reconnaissance de tel ou tel épitope de l'antigène de 28 kDa est spécifique d'espèce.

### EXEMPLE 3 - CONTROLE DE LA RECONNAISSANCE DU PEPTIDE ANTIGENIQUE CONFORME A LA PRESENTE INVENTION PAR LES ANTICORPS MONOCLONAUX

A. La recherche d'un épitope peptidique de taille restreinte parmi les produits de protéolyse de l'antigène de 28 KD, a permis de révéler un fragment de 8 KD reconnu spécifiquement par l'anticorps monoclonal d'isotype IgG2a, obtenu à partir de l'hybridome W 2 AD 12.
L'activité cytotoxique (illustrée dans le Tableau I ci-après) de cet anticorps monoclonal dans des tests in vitro impliquant des éosinophiles, suggère l'importance du ou des épitopes portés par ce peptide de 8 KD.

**TABLEAU I**

| Cytotoxicité dépendante d'éosinophiles Etude de l'anticorps monoclonal W 2 AD 12 | |
|---|---|
| Source d'anticorps ^{a} | Pourcentage de cyto- ^{b} toxicité ± S.D. |
| - Sérum de rat porteur de la tumeur sous-cutanée d'hybride W 2 AD 12 | 66,9 ± 26,0 |
| - Sérum de rat porteur de la tumeur sous-cutanée des cellules IR983F | 7,3 ± 1,06 |
| - Sérum de rat immunisé par la protéine 28 kDa | 69,9 ± 22,9 |
| - Sérum de rat infecté depuis 4 semaines par S. mansoni | 74,3 ± 23,5 |
| - Sérum de rat sain | 5,0 ± 4,2 |

Les tests de cytotoxicité sont réalisés selon les conditions décrites par Capron et Coll. [Eur. J. Immunol., 8, 127-133, 1978].
a) Les schistosomules sont présensibilisés pendant 18 heures, en présence de 100 µl des différents sérums (dilution finale 1/16) chauffés 1 heure à 56°C,
b) Le pourcentage de cytotoxicité (moyenne ± écart-type de la moyenne) est mesuré après 48 heures d'incubation des schistosomules sensibilisés en présence de cellules péritonéales de rat sain (rapport cellules effectrices/cible = 6000/1).
Le sérum de lapin anti-28 KD ne reconnait pas le ou les épitopes portés par ce peptide, mais reconnait un autre peptide, de 6 KD.
L'anticorps monoclonal de W 2 AD 12 reconnait les deux peptides du groupe d'antigènes de 28 KD.
B. L'anticorps monoclonal d'isotype IgM, obtenu à partir de l'hybridome M 5 BD 9 ne reconnait qu'une seule des deux protéines du groupe à 28 kDa.

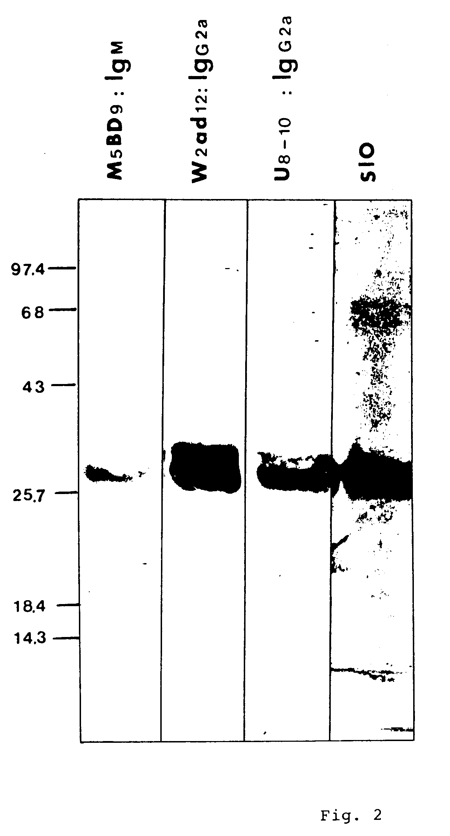

La Figure 2 annexée montre les résultats de "Western blotting" du groupe d'antigènes de 28 kDa conforme à l'invention avec des anticorps monoclonaux de M 5 BD 9, de W 2 AD 12 et avec du sérum polyclonal de lapin, tels que relatés ci-dessus.

## Revendications (Revendications pour l'(les) Etat(s) contractant(s) suivant(s): BE, CH, DE, FR, GB, IT, LI, LU, NL, SE)

1. Procédé d'isolement d'un peptide portant au moins un épitope, par protéolyse, caractérisé en ce que l'on soumet les protéines du groupe d'antigènes de 28 kD de *S*. *mansoni* à l'action de la protéase V8 en tampon Tris-HCl pH 6,8 contenant du SDS, en ce que la réaction de protéolyse est arrêtée par addition d'un mélange de 2-mercaptoéthanol et de SDS, puis on porte à ébullition à 100°C pendant un temps bref, pour recueillir un peptide dont on vérifie l'activité antigénique à l'aide des anticorps d'un sérum polyclonal ou d'anticorps monoclonaux.

2. Procédé selon la Revendication 1, caractérisé en ce que la digestion enzymatique des protéines du groupes d'antigènes de 28 kD par la protéase V8 est réalisée pendant 30 minutes à 30°C.

3. Procédé selon l'une quelconque des Revendications 1 et 2, caractérisé en ce que le mélange utilisé pour arrêter la réaction de protéolyse comprend du 2-mercaptoéthanol à raison de 1 µl pour 3 µl de SDS à 10%.

4. Procédé selon l'une quelconque des Revendications 1 à 3, caractérisé en ce que le tampon Tris-HCl dans lequel sont dissous les protéines du groupe d'antigènes de 28 kD est un tampon 100 mM à 125 mM et contient de 0,08 à 0,12% de SDS.

5. Procédé selon l'une quelconque des Revendications 1 à 4, caractérisé en ce que la vérification de l'activité antigénique des peptides isolés par protéolyse, qui est réalisée par "Western blotting" à l'aide d'un sérum polyclonal de rat, de lapin ou analogue, anti-28 kD, révèle, à l'aide d'anticorps marqués par la peroxydase, dirigés contre des immunoglobulines de rat, de lapin, de souris ou analogues, un peptide de 6kD.

6. Procédé selon l'une quelconque des Revendications 1 à 4, caractérisé en ce que la vérification de l'activité antigénique du peptide isolé par protéolyse, qui est réalisée par "Westerm blotting" à l'aide d'un anticorps monoclonal anti-28 kD révèle un peptide de 8 kD.

7. Fragment peptidique, caractérisé en ce qu'il est isolé à partir des protéines du groupe d'antigènes de 28 kD de *S*. *mansoni*, par protéolyse ménagée, selon la Revendication 5, en ce qu'il présente un poids moléculaire de 6 kD et en ce qu'il est reconnu par du sérum polyclonal de lapin, de rat ou analogue, anti-28 kD.

8. Hybridome dénommé W2AD12, déposé à la CNCM le 9 Mai 1986 sous le n^{o} I-553, qui fournit des anticorps monoclonaux d'isotype IgG2a, reconnaissant le groupe d'antigènes de 28 kD de *S*. *mansoni*.

9. Fragment peptidique, caractérisé en ce qu'il est isolé à partir des protéines du groupe d'antigènes de 28 kD de *S*. *mansoni*, par protéolyse ménagée, selon la Revendication 6, en ce qu'il présente un poids moléculaire de 8 kD et en ce qu'il est reconnu par l'anticorps monoclonal anti-28 kD, d'isotype IgG2a, produit par l'hybridome W2AD12 défini dans la Revendication 8.

10. Composition vaccinante contre *S*. *mansoni*, caractérisée en ce qu'elle contient au moins un fragment peptidique isolé des protéines du groupe d'antigène de 28 kD de *S*. *mansoni* par protéolyse ménagéé, selon l'une quelconque des Revendications 1 à 4, associé à un véhicule approprié.

11. Composition vaccinante selon la Revendication 10, caractérisée en ce qu'elle contient au moins un fragment peptidique de 6 kD et/ou de 8 kD, selon l'une quelconque des Revendications 7 ou 9, associé à un véhicule approprié.

12. Composition vaccinante selon l'une quelconque des Revendications 10 et 11, caractérisée en ce qu'elle contient au moins un fragment peptidique isolé de *S*. *mansoni* par protéolyse ménagée, associé à l'épitope de l'antigène 38 kD de *S*. *mansoni*, ainsi qu'à un véhicule approprié.

## Revendications (Revendications pour l'(les) Etat(s) contractant(s) suivant(s): AT, GR)

1. Procédé d'isolement d'un peptide portant au moins un épitope, par protéolyse, caractérisé en ce que l'on soumet les protéines du groupe d'antigènes de 28 kD de *S*. *mansoni* à l'action de la protéase V8 en tampon Tris-HCl pH 6,8 contenant du SDS, en ce que la réaction de protéolyse est arrêtée par addition d'un mélange de 2-mercaptoéthanol et de SDS, puis on porte à ébullition à 100°C pendant un temps bref, pour recueillir un peptide dont on vérifie l'activité antigénique à l'aide des anticorps d'un sérum polyclonal ou d'anticorps monoclonaux.

2. Procédé selon la Revendication 1, caractérisé en ce que la digestion enzymatique des protéines du groupes d'antigènes de 28 kD par la protéase V8 est réalisée pendant 30 minutes à 30°C.

3. Procédé selon l'une quelconque des Revendications 1 et 2, caractérisé en ce que le mélange utilisé pour arrêter la réaction de protéolyse comprend du 2-mercaptoéthanol à raison de 1 µl pour 3 µl de SDS à 10%.

4. Procédé selon l'une quelconque des Revendications 1 à 3, caractérisé en ce que le tampon Tris-HCl dans lequel sont dissous les protéines du groupe d'antigènes de 28 kD est un tampon 100 mM à 125 mM et contient de 0,08 à 0,12% de SDS.

5. Procédé selon l'une quelconque des Revendications 1 à 4, caractérisé en ce que la vérification de l'activité antigénique des peptides isolés par protéolyse, qui est réalisée par "Western blotting" à l'aide d'un sérum polyclonal de rat, de lapin ou analogue, anti-28 kD, révèle, à l'aide d'anticorps marqués par la peroxydase, dirigés contre des immunoglobulines de rat, de lapin, de souris ou analogues, un peptide de 6kD.

6. Procédé selon l'une quelconque des Revendications 1 à 4, caractérisé en ce que la vérification de l'activité antigénique du peptide isolé par protéolyse, qui est réalisée par "Westerm blotting" à l'aide d'un anticorps monoclonal anti-28 kD révèle un peptide de 8 kD.

7. Procédé d'obtention d'un fragment peptidique, caractérisé en ce que l'on isole à partir des protéines du groupe d'antigènes de 28 kD de *S*. *mansoni*, par protéolyse ménagée, selon la Revendication 5, un fragment qui présente un poids moléculaire de 6 kD et qui est reconnu par du sérum polyclonal de lapin, de rat ou analogue, anti-28 kD.

8. Procédé d'obtention d'anticorps monoclonaux d'isotype IgG2a, qui reconnaissent le groupe d'antigènes de 28 kD de *S*. *mansoni*, caractérisé en ce que lesdits anticorps monoclonaux sont obtenus à partir d'un Hybridome dénommé W2AD12, déposé à la CNCM le 9 Mai 1986 sous le n^{o} I-553.

9. Procédé d'obtention d'un fragment peptidique, caractérisé en ce que l'on isole à partir des protéines du groupe d'antigènes de 28 kD de *S*. *mansoni*, par protéolyse ménagée, selon la Revendication 6, un fragment qui présente un poids moléculaire de 8 kD et qui est reconnu par l'anticorps monoclonal anti-28 kD, d'isotype IgG2a, produit par l'hybridome W2AD12 défini dans la Revendication 8.

10. Procédé de préparation d'une composition vaccinante contre *S*. *mansoni*, caractérisée en ce que l'on associe au moins un fragment peptidique isolé des protéines du groupe d'antigène de 28 kD de *S*. *mansoni* par protéolyse ménagée, selon l'une quelconque des Revendications 1 à 4 associé à un véhicule approprié.

11. Procédé de préparation d'une composition vaccinante selon la Revendication 10, caractérisée en ce que l'on associe au moins un fragment peptidique de 6 kD et/ou de 8 kD, obtenu par procédé selon l'une quelconque des Revendications 7 ou 9, à un véhicule approprié.

12. Procédé de préparation d'une composition vaccinante selon l'une quelconque des Revendications 10 et 11, caractérisée en ce que l'on associe au moins un fragment peptidique isolé de 28 kD de *S*. *mansoni* par protéolyse ménagée, à l'épitope de l'antigène 38 kD de *S*. *mansoni*, ainsi qu'à un véhicule approprié.

## Claims (Claims for the following Contracting State(s): BE, CH, DE, FR, GB, IT, LI, LU, NL, SE)

1. A process for isolating a peptide carrying at least one epitope, by proteolysis, characterized in that the proteins of the group of 28 kD antigens of S. mansoni are subjected to the action of V8 protease in a tris-HCl pH 6.8 buffer containing SDS, whereafter the proteolysis reaction is stopped by the addition of a mixture of 2-mercaptoethanol and SDS, whereafter boiling is briefly performed at 100°C, to recover a peptide whose antigen activity is verified by means of antibodies of a polyclonal serum or of monoclonal antibodies.

2. A process according to claim 1, characterized in that the enzymatic digestion of the proteins of the groups of 28 kD antigens by V8 protease is performed for 30 minutes at 30°C.

3. A process according to either of claims 1 and 2, characterized in that the mixture used to stop the proteolysis reaction comprises 2-mercaptoethanol at the rate of 1 µl to 3 µl of 10% SDS.

4. A process according to any of claims 1 to 3, characterized in that the tris-HCl buffer in which the proteins of the group of 28 kD antigens are dissolved is a 100 mM to 125 mM buffer and contains from 0.08 to 0.12% SDS.

5. A process according to any of claims 1 to 4, characterized in that the verification of the antigenic activity of the peptides isolated by proteolysis, which is performed by "Western blotting", using a polyclonal rat, rabbit or similar anti-28 kD serum, reveals by means of antibodies tagged by the peroxydase and directed against rat, rabbit, mouse or similar immunoglobulins, a 6 kD peptide.

6. A process according to any of claims 1 to 4, characterized in that the verification of the antigenic activity of the peptide isolated by proteolysis, which is performed by "Western blotting" using a monoclonal anti-28 kD antibody, reveals an 8 kD peptide.

7. A peptide fragment, characterized in that it is isolated from the proteins of the group of 28 kD antigens of S. mansoni by managed proteolysis, according to claim 5, it has a molecular weight of 6 kD, and it is recognized by a polyclonal rabbit, rat or similar anti-28 kD serum.

8. A hybridome known as W2AD12, filed with the CNCM on 9 May 1986 under No. I-553, which supplies monoclonal IgG2a isotype antibodies recognizing the group of 28 kD antigens of S. mansoni.

9. A peptide fragment, characterized in that it is isolated from the proteins of the group of 28 kD antigens of S. mansoni by managed proteolysis, according to claim 6, it has a molecular weight of 8 kD, and it is recognized by the monoclonal IgG2a isotype anti-28 kD antibody produced by the W2AD12 hybridome defined in claim 8.

10. A vaccinating composition against S. mansoni, characterized in that it contains at least one peptide fragment isolated from the proteins of the group of 28 kD antigens of S. mansoni by managed proteolysis, according to any of claims 1 to 9, associated with a suitable vehicle.

11. A vaccinating composition according to claim 10, characterized in that it contains at least one 6 kD and/or 8 kD peptide fragment, associated to either of claims 7 or 9, associated with a suitable vehicle.

12. A vaccinating composition according to any of claims 10 and 11, characterized in that it contains at least one isolated peptide fragment of S. mansoni obtained by managed proteolysis, associated with the epitope of the 38 kD antigen of S. mansoni, and also with a suitable vehicle.

## Claims (Claims for the following Contracting State(s): AT, GR, ES)

1. A process for isolating a peptide carrying at least one epitope, by proteolysis, characterized in that the proteins of the group of 28 kD antigens of S. mansoni are subjected to the action of V8 protease in a tris-HCl pH 6.8 buffer containing SDS, whereafter the proteolysis reaction is stopped by the addition of a mixture of 2-mercaptoethanol and SDS, whereafter boiling is briefly performed at 100°C, to recover a peptide whose antigen activity is verified by means of antibodies of a polyclonal serum or of monoclonal antibodies.

2. A process according to claim 1, characterized in that the enzymatic digestion of the proteins of the groups of 28 kD antigens by V8 protease is performed for 30 minutes at 30°C.

3. A process according to either of claims 1 and 2, characterized in that the mixture used to stop the proteolysis reaction comprises 2-mercaptoethanol at the rate of 1 µl to 3 µl of 10% SDS.

4. A process according to any of claims 1 to 3, characterized in that the tris-HCl buffer in which the proteins of the group of 28 kD antigens are dissolved is a 100 mM to 125 mM buffer and contains from 0.08 to 0.12% SDS.

5. A process according to any of claims 1 to 4, characterized in that the verification of the antigenic activity of the peptides isolated by proteolysis, which is performed by "Western blotting", using a polyclonal rat, rabbit or similar anti-28 kD serum, reveals by means of antibodies tagged by the peroxydase and directed against rat, rabbit, mouse or similar immunoglobulins, a 6 kD peptide.

6. A process according to any of claims 1 to 4, characterized in that the verification of the antigenic activity of the peptide isolated by proteolysis, which is performed by "Western blotting" using a monoclonal anti-28 kD antibody, reveals an 8 kD peptide.

7. A peptide fragment, characterized in that it is isolated from the proteins of the group of 28 kD antigens of S. mansoni by managed proteolysis, according to claim 5, it has a molecular weight of 6 kD, and it is recognized by a polyclonal rabbit, rat or similar anti-28 kD serum.

8. A process for obtaining monoclonal IgG2a isotype antibodies which recognize the group of 28 kD antigens of S. mansoni, characterized in that said monoclonal antibodies are obtained from a hybridome known as W2AD12, filed with the CNCM on 9 May 1986 under No. I-553.

9. A process for obtaining a peptide fragment, characterized in that there is isolated from the proteins of the group of 28 kD antigens of S. mansoni, by managed proteolysis according to claim 6, a fragment which has a molecular weight of 8 kD, and which is recognized by the monoclonal IgG2a isotype anti-28 kD antibody produced by the W2AD12 hybridome defined in claim 8.

10. A process for the preparation of a vaccinating composition against S. mansoni, characterized in that there is associated with a suitable vehicle at least one peptide fragment isolated from the proteins of the group of 28 kD antigens of S. mansoni, by managed proteolysis according to any of claims 1 to 4, associated with a suitable vehicle.

11. A process for the preparation of a vaccinating composition according to claim 10, characterized in that there is associated with a suitable vehicle at least one 6 kD and/or 8 kD peptide fragment obtained by the process according to either of claims 7 or 9.

12. A process for the preparation of a vaccinating composition according to either of claims 10 and 11, characterized in that there is associated at least one peptide fragment isolated from 28 kD of S. mansoni by managed proteolysis with the epitope of the 38 kD antigens of S. mansoni and also with a suitable vehicle.

## Patentansprüche (Patentansprüche für folgende(n) Vertragsstaat(en): BE, CH, DE, FR, GB, IT, LI, LU, NL, SE)

1. Verfahren zur Isolierung eines mindestens ein Epitop tragenden Peptids durch Proteolyse, dadurch **gekennzeichnet**, daß man die Proteine der Gruppe der Antigene mit 28 kD von S. mansoni der Wirkung der Protease V8 in einem Tris-HCl Puffer mit pH 6,8, der SDS enthält, unterwirft, man die proteolytische Reaktion durch Zugabe eines Gemisches aus 2-Mercaptoethanol und SDS stoppt, anschließend das Gemisch für kurze Zeit zum Sieden auf 100°C bringt, um ein Peptid zu gewinnen, dessen antigene Aktivität man mit Hilfe von Antikörpern eines polyclonalen Serums oder von monoclonalen Antikörpern verifiziert.

2. Verfahren nach Anspruch 1, dadurch **gekennzeichnet**, daß man den enzymatischen Abbau der Proteine der Gruppen der Antigene mit 28 kD durch die Protease V8 während 30 Minuten bei 30°C durchführt.

3. Verfahren nach einem der Ansprüche 1 und 2, dadurch **gekennzeichnet**, daß das Gemisch, das zum Stoppen der proteolytischen Reaktion verwendet wird, 2-Mercaptoethanol im Verhältnis von 1 µl auf 3 µl 10%iges SDS enthält.

4. Verfahren nach einem der Ansprüche 1 bis 3, dadurch **gekennzeichnet**, daß der Tris-HCl Puffer, in dem die Proteine der Gruppe der Antigene mit 28 kD gelöst sind, ein 100 mM bis 125 mM Puffer ist und 0,08 bis 0,12 % SDS enthält.

5. Verfahren nach einem der Ansprüche 1 bis 4, dadurch **gekennzeichnet**, daß die Verifizierung der antigenen Aktivität der durch Proteolyse isolierten Peptide, die durch Western Blotting mit Hilfe eines polyclonalen Anti-28 kD-Serums der Ratte, des Kaninchens oder eines Analogen davon durchgeführt wird, mit Hilfe von durch Peroxidase markierten Antikörpern gegen die Immunglobuline der Ratte, des Kaninchens, der Maus oder Analogen ein 6 kD-Peptid nachweist.

6. Verfahren nach einem der Ansprüche 1 bis 4, dadurch **gekennzeichnet**, daß die Verifizierung der antigenen Aktivität des durch Proteolyse isolierten Peptids die mittels Western Blotting mit Hilfe eines monoclonalen Anti-28 kD-Antikörpers durchgeführt wird, ein 8 kD-Peptid nachweist.

7. Peptidfragment, dadurch **gekennzeichnet**, daß es aus Proteinen der Gruppe der Antigene mit 28 kD von S. mansoni durch schonende Proteolyse nach Anspruch 5 isoliert wird, daß es ein Molekulargewicht von 6 kD besitzt und daß es von einem polyclonalen Anti-28 kD-Serum eines Kaninchens, einer Ratte oder eines Analogen davon erkannt wird.

8. Hybridom mit der Bezeichnung W2AD12, hinterlegt bei der CNCM am 9. Mai 1986 unter der Nummer I-553, dadurch **gekennzeichnet**, daß es monoclonale Antikörper vom Isotyp IgG2a, welche die Gruppe der Antigene mit 28 kD von S. mansoni erkennen, erzeugt.

9. Peptidfragment, dadurch **gekennzeichnet**, daß es aus Proteinen der Gruppe der Antigene mit 28 kD von S. mansoni durch schonende Proteolyse nach Anspruch 6 isoliert wird, daß es ein Molekulargewicht von 8 kD besitzt und daß es von monoclonalen Anti-28-kD-Antikörpern des Isotyps IgG2a, erzeugt von dem Hybridom W2AD12, definiert in Anspruch 8, erkannt wird.

10. Impfzusammensetzung gegen S. mansoni, dadurch **gekennzeichnet**, daß sie mindestens ein aus Proteinen der Antigengruppe mit 28 kD von S. mansoni durch schonende Proteolyse isoliertes Peptid nach einem der Ansprüche 1 bis 4 zusammen mit einem geeigneten Träger enthält.

11. Impfzusammensetzung nach Anspruch 10, dadurch **gekennzeichnet**, daß sie mindestens ein Peptidfragment von 6 kD und/oder 8 kD nach einem der Ansprüche 7 oder 9 zusammen mit einem geeigneten Träger enthält.

12. Impfzusammensetzung nach einem der Ansprüche 10 und 11, dadurch **gekennzeichnet**, daß sie mindestens ein aus S. mansoni durch schonende Proteolyse isoliertes Peptidfragment, das mit dem Epitop des 28 kD-Antigens von S. mansoni assoziiert ist, ebenso wie einen geeigneten Träger enthält.

## Patentansprüche (Patentansprüche für folgende(n) Vertragsstaat(en): AT, ES, GR)

1. Verfahren zur Isolierung eines mindestens ein Epitop tragenden Peptids durch Proteolyse, dadurch **gekennzeichnet**, daß man die Proteine der Gruppe der Antigene mit 28 kD von S. mansoni der Wirkung der Protease V8 in einem Tris-HCl Puffer mit pH 6,8, der SDS enthält, unterwirft, man die proteolytische Reaktion durch Zugabe eines Gemisches aus 2-Mercaptoethanol und SDS stoppt, anschließend das Gemisch für kurze Zeit zum Sieden auf 100°C bringt, um ein Peptid zu gewinnen, dessen antigene Aktivität man mit Hilfe von Antikörpern eines polyclonalen Serums oder von monoclonalen Antikörpern verifiziert.

2. Verfahren nach Anspruch 1, dadurch **gekennzeichnet**, daß man den enzymatischen Abbau der Proteine der Gruppen der Antigene mit 28 kD durch die Protease V8 während 30 Minuten bei 30°C durchführt.

3. Verfahren nach einem der Ansprüche 1 und 2, dadurch **gekennzeichnet**, daß das Gemisch, das zum Stoppen der proteolytischen Reaktion verwendet wird, 2-Mercaptoethanol im Verhältnis von 1 µl auf 3 µl 10%iges SDS enthält.

4. Verfahren nach einem der Ansprüche 1 bis 3, dadurch **gekennzeichnet**, daß der Tris-HCl Puffer, in dem die Proteine der Gruppe der Antigene mit 28 kD gelöst sind, ein 100 mM bis 125 mM Puffer ist und 0,08 bis 0,12 % SDS enthält.

5. Verfahren nach einem der Ansprüche 1 bis 4, dadurch **gekennzeichnet**, daß die Verifizierung der antigenen Aktivität der durch Proteolyse isolierten Peptide, die durch Western Blotting mit Hilfe eines polyclonalen Anti-28 kD-Serums der Ratte, des Kaninchens oder eines Analogen davon durchgeführt wird, mit Hilfe von durch Peroxidase markierten Antikörpern gegen die Immunglobuline der Ratte, des Kaninchens, der Maus oder Analogen ein 6 kD-Peptid nachweist.

6. Verfahren nach einem der Ansprüche 1 bis 4, dadurch **gekennzeichnet**, daß die Verifizierung der antigenen Aktivität des durch Proteolyse isolierten Peptids die mittels Western Blotting mit Hilfe eines monoclonalen Anti-28 kD-Antikörpers durchgeführt wird, ein 8 kD-Peptid nachweist.

7. Peptidfragment, dadurch **gekennzeichnet**, daß es aus Proteinen der Gruppe der Antigene mit 28 kD von S. mansoni durch schonende Proteolyse nach Anspruch 5 isoliert wird, daß es ein Molekulargewicht von 6 kD besitzt und daß es von einem polyclonalen Anti-28 kD-Serum eines Kaninchens, einer Ratte oder eines Analogen davon erkannt wird.

8. Verfahren zum Erhalt monoclonaler Antikörper vom Isotyp IgG2a, die die Gruppe der Antigene mit 28 kD von S. mansoni erkennen, dadurch **gekennzeichnet**, daß die monoclonalen Antikörper aus einem Hybridom mit der Bezeichnung W2AD12, hinterlegt bei der CNCM am 9. Mai 1986, unter der Nr. I-553 erhalten werden.

9. Verfahren zum Erhalt eines Peptidfragments, dadurch **gekennzeichnet**, daß man aus Proteinen der Gruppe der Antigene mit 28 kD von S. mansoni durch schonende Proteolyse nach Anspruch 6 ein Fragment isoliert, das ein Molekulargewicht von 8 kD besitzt und das von einem monoclonalen Anti-28 kD-Antikörper des Isotyps IgG2a, erzeugt von dem Hybriom W2AD12, wie in Anspruch 8 definiert, erkannt wird.

10. Verfahren zur Herstellung einer Impfzusammensetzung gegen S. mansoni, dadurch **gekennzeichnet**, daß man mindestens ein Peptidfragment, isoliert aus Proteinen der Gruppe der Antigene mit 28 kD von S. mansoni durch schonende Proteolyse nach einem der Ansprüche 1 bis 4, zusammen mit einem geeigneten Träger vermischt.

11. Verfahren zur Herstellung einer Impfzusammensetzung nach Anspruch 10, dadurch **gekennzeichnet**, daß man mindestens ein Peptidfragment von 6 kD und/oder 8 kD, erhalten nach einem Verfahren nach einem der Ansprüche 7 oder 9, mit einem geeigneten Träger vermischt.

12. Verfahren zur Herstellung einer Impfzusammensetzung nach einem der Ansprüche 10 und 11, dadurch **gekennzeichnet**, daß man mindestens ein Peptidfragment, isoliert aus 28 kD von S. mansoni durch schonende Proteolyse, mit dem Epitop des Antigens mit 38 kD von S. mansoni zusammen mit einem geeigneten Träger vermischt.
